Europäisches Patentamt

European Patent Office

Office européen des brevets

(11) Publication number: **0 238 893 B1**

## (12) EUROPEAN PATENT SPECIFICATION

(45) Date of publication of patent specification: **28.10.92**

(21) Application number: **87102923.7**

(22) Date of filing: **02.03.87**

(51) Int. Cl.⁵: **C12N 15/70**, A61K 39/23,
C12Q 1/70, G01N 33/569,
C07K 15/00

(54) Recombinant parvovirus RA-1 products and methods of use thereof.

(30) Priority: **20.03.86 US 841905**

(43) Date of publication of application:
**30.09.87 Bulletin 87/40**

(45) Publication of the grant of the patent:
**28.10.92 Bulletin 92/44**

(84) Designated Contracting States:
**BE DE FR IT**

(56) References cited:

**SCIENCE, vol. 223, 30th March 1984, pages
1425-1428; R.W. SIMPSON et al.: "Association
of Parvoviruses with rheumatoid arthritis of
humans"**

**SCIENCE, vol. 226, 7th December 1984,
pages 1161-1165; S.F. COTMORE et al.:
"Characterization and molecular cloning of a
human parvovirus genome"**

(73) Proprietor: **ABBOTT LABORATORIES
14th Street and Sheridan Road North St
North Chicago, Illinois 60064(US)**

(72) Inventor: **Simpson, Robert Wayne
261 Amwell Road
Neshanic New Jersey 08853(US)**
Inventor: **VanLeeuwen, Donald Hugh
1936 Poplar Street
Waukegan Illinois 60087(US)**
Inventor: **Smith, Robert Eldon
36567 N. Grandwood Drive
Gurnee Illinois 60031(US)**
Inventor: **Lawrie, Dawson Keiss
1114-35th Street
Downers Grove Illinois 60515(US)**

(74) Representative: **Modiano, Guido et al
c/o Modiano & Associati S.r.l. Via Meravigli,
16
I-20123 Milano(IT)**

BIOTECHNOLOGY, vol. 5, no. 10, October 1987, pages 1077-1080, New York, US; W.P. SISK et al.: "Expression of human parvovirus B19 structural protein in E. Coli and detection of antiviral antibodies in human serum"

ABSTRACTS OF THE ANNUAL MEETING OF THE AMERICAN SOCIETY FOR MICROBIOLOGY, May 1986, vol. 86, no. 0, page 316, no. S-42, American Society for Microbiology, US; D.K. LAWRIE et al.: "Cloning and in-vitro expression of parvovirus RA-1 DNA in Escherichia-Coli"

THE LANCET, 23rd February 1985, pages 422-425; D.M. REID et al.: "Human parvovirus-associated arthritis: a clinical and laboratory description"

VIROLOGY, vol. 134, no. 1, 1984, pages 64-71, Academic Press, Inc.; B. GEORG-FRIES et al.: "Analysis of proteins, helper dependence, and seroepidemiology of a new human parvovirus"

JOURNAL OF MEDICAL VIROLOGY, vol. 15, no. 2, 1985, pages 173-181, Alan R. Liss, Inc.; J.P. CLEWLEY: "Detection of human paravovirus using a molecularly cloned probe"

**Description**

BACKGROUND OF THE INVENTION

Field of the Invention

This invention relates generally to the recombinant deoxyribonucleic acid (DNA) products of Parvovirus RA-1, and particularly to its cloned DNA and expressed gene products. Such products may be particularly useful as diagnostic reagents for the determination of human connective tissue disorders, such as rheumatoid arthritis.

Background Art

The Parvoviridae is a family of viruses with linear, single-stranded DNA genomes 4.5 to 5.2 kilobases in length. Each terminus of the genome ends in a characteristic "hairpin loop" structure due to the presence of pallindromic DNA sequences. In addition to DNA, mature virions contain 3 or 4 polypeptides all of which share some sequence redundancy. Viral particles appear by electron microscopy to be very small (18-26 nm in diameter), non-enveloped, and have icosahedral symmetry.

The parvoviruses of vertebrates are broadly categorized into autonomous and defective classes. Autonomous parvoviruses (genus Parvovirus) are capable of sustaining their replication in host cells, but require one or more cellular functions. The defective parvoviruses (genus Dependovirus) require coinfection by a helper virus to provide additional functions needed for replication.

The best studied autonomous parvovirus is the minute virus of mice (MVM). The genome of MVM has been cloned in E. coli, and sequenced in its entirety. Ribonucleic acid (RNA) transcripts have been mapped to the MVM genome revealing that three messenger RNA (mRNA) species are present (R1, R2 and R3) which share common sequences. All viral mRNAs are spliced and polyadenylated.

Analysis of viral-coded proteins synthesized in parvovirus-infected cells has shown that four proteins are produced. Two structural proteins (VP1 and VP2), which make up the parvovirus capsid, map predominantly to the 5'-half of the genome. Two other proteins, which appear to be non-structural (NS1 and NS2), map to the 3'-half of the genome. In addition to coding regions, the MVM genome has been shown to contain pallindromic sequences at the 5' and 3'-termini which are required for replication.

Other autonomous animal parvoviruses have been studied in rat (H-1), dog (CPV), cat (FPLV), cow (BPV), pig (PPV) and mink (ADV). All have structural features generally consistent with those observed for MVM, although there are specific differences in DNA sequence and amino acid content.

The gene expression of several animal autonomous parvoviruses has been explored in mammalian cells and in E. coli. The cloned genome of MVM has been transfected into mammalian cells giving rise to infectious progeny at low efficiency. Expression of MVM DNA in mammalian cells using a eucaryotic expression vector resulted in the synthesis of VP1 and VP2 structural proteins. However, the non-structural proteins NS1 and NS2) have not been detected.

Cloned mink parvovirus (ADV) DNA has been shown to be expressed in E. coli yielding proteins unlike those observed during virus infection. DNA regions of the canine parvovirus (CPV) and porcine parvovirus (PPV) genome have also been expressed in E. coli as fusion proteins.

The first parvovirus associated with a human disease was B19. This virus has been clinically associated with Fifth Disease in children, a transient arthritis in adults, and erythrocyte aplasia in chronic hemolytic anemias of adults. See Anderson, M.J., J. of Hygiene 89:1-8 (1982); Anderson, M.J., et al., Lancet i:1378 (1983); White, D.G., et al., Lancet i:419-421 (1985); and Anderson, M.J., et al., Archives of Virology 82:137-148 (1984). The viral DNA of B19 has been recently cloned from a clinical isolate of the virus. See Cotmore, S.F., et al., Science 226:1161-1165 (1984).

SUMMARY OF THE INVENTION

This invention involves a novel utilization of the parvovirus RA-1, which has been isolated from the synovial fluid of a human patient with chronic rheumatoid arthritis. This virus has been propagated in neonatal mice, where it has been determined to elicit permanent crippling of limbs and other structural deformities. In accordance with the present invention, a novel cloning of parvovirus RA-1 DNA, derived from RA-1 virus particles isolated from the brains of such infected neonatal mice, has been accomplished. In particular, the viral DNA isolated in single-stranded form has been made double-stranded, using T4 DNA polymerase, and cloned (after removal of the terminal pallindromic sequences) in a plasmid vector in bacteria, e.g., E. coli. The DNA has been characterized by restriction endonuclease mapping and shown to be unique from that of any previously described parvovirus.

Further, cloned parvovirus RA-1 DNA has been used, according to the instant invention, as a DNA diagnostic probe to screen clinical samples of synovial fluid from patients diagnosed as having rheumatoid arthritis, demonstrating the utility of parvovirus RA-1 DNA as a useful diagnostic probe for rheumatoid arthritis.

Moreover, according to preferred embodiments of the present invention, parvovirus RA-1 cloned DNA has been expressed in vitro in an E. coli cell-free system and in vivo using the protease-deficient E. coli strain WM6. In addition, the present invention has been found to enable the construction of defined subclones for expression of parvovirus RA-1 structural genes. The consequently expressed antigens from E. coli, or from other expression systems such as yeast, mammalian cells or nuclear polyhedrosis virus-insect cells, also can be seen to have utility as diagnostic probes for detection of antibodies to parvovirus RA-1 which may be present in clinical samples of patients with rheumatoid arthritis, thereby providing efficacious assays for this crippling condition.

DESCRIPTION OF THE DRAWINGS

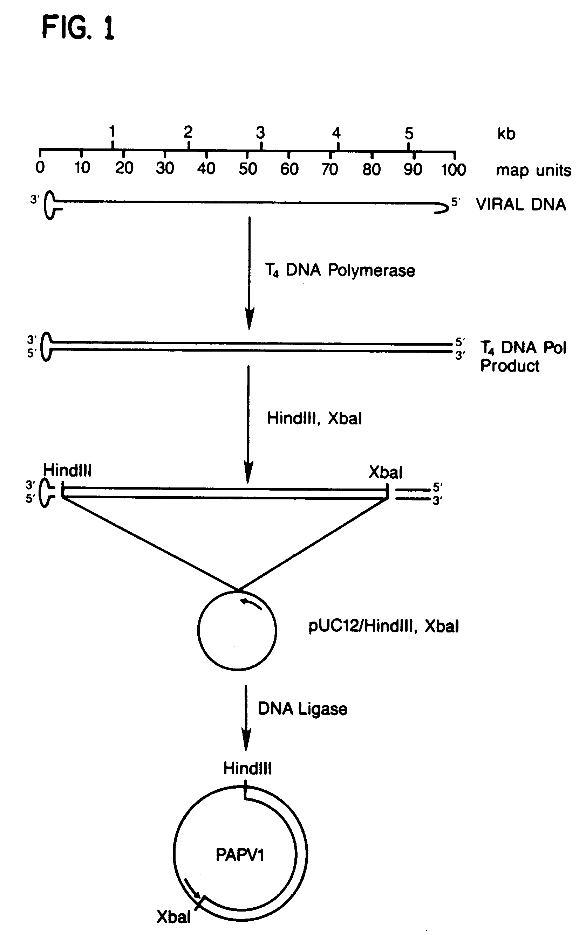

Fig. 1 is a flow diagram showing the in vitro synthesis of parvovirus RA-1 double-stranded DNA from single-stranded genomic DNA using T4 DNA polymerase. The product, double-stranded DNA, was then digested with restriction endonucleases Hind III and Xba I (restriction sites 5 to 85 map units) and ligated to pUC12 to generate pAPV1.

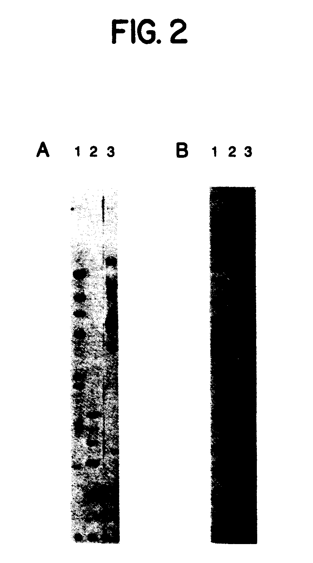

Fig. 2 illustrates the size of the RA-1 product double-stranded DNA on a neutral agarose gel (0.7% agarose, 40 millimolar (mM) Tris, 5 mM sodium acetate, pH 7.9, 2mM EDTA) (Panel A); and on an alkaline agarose gel (1% agarose, 60mM NaOH, 2mM EDTA) (Panel B). The migration of lambda, Hind III size markers (lane 1); Phi X, Hae III size markers (lane 2); and RA-1 double-stranded DNA (lane 3) are shown in both panels.

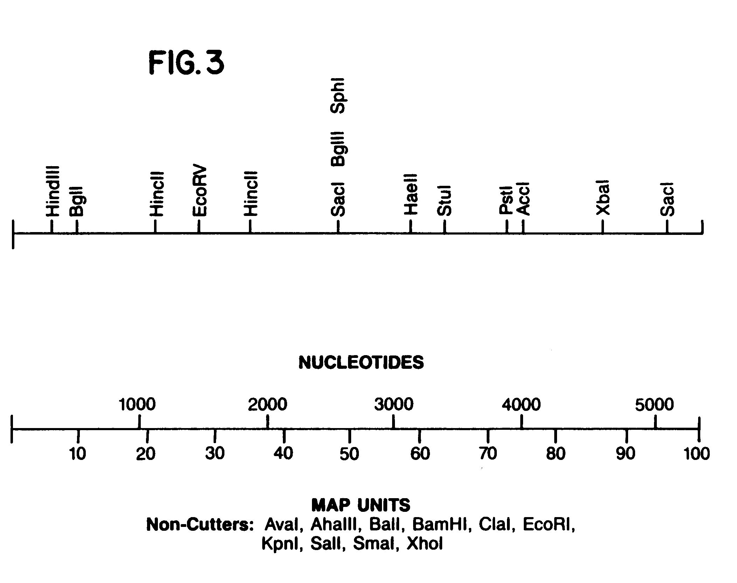

Fig. 3 shows a restriction map of the parvovirus RA-1 DNA product of T4 DNA polymerase, produced by compiling the locations of sites within the DNA digested by restriction endonucleases Acc I, Bgl I, Bgl II, Eco RV, Hae II, Hinc II, Hind III, Pst I, Sac I, Sph I, Stu I and Xba I. In addition, restriction sites were found to be absent for restriction endonucleases Ava I, Aha III, Bal I, Bam HI, Cla I, Eco RI, Kpn I, Sal I, Sma I and Xho I.

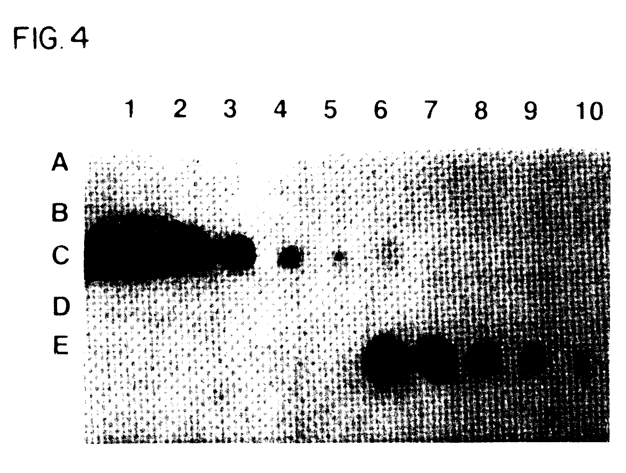

Fig. 4 shows the hybridization of radiolabeled cloned RA-1 DNA to DNA contained in tissue samples from normal and RA-1 infected mice. Tissues assayed were Hirt extracts of normal mouse brain (A1-A5); normal mouse liver (A6-A10); normal mouse spleen (B1-B5); normal mouse blood (B6-B10); RA-1 infected mouse brain (C1-C5); RA-1 infected mouse liver (C6-C10); RA-1 infected mouse spleen (D1-D5); RA-1 infected mouse blood (D6-D10); a phenol extract of a Hirt supernate of normal mouse brain (E1-E5); a phenol extract of a Hirt supernate of RA-1 infected mouse brain (E6-E10). For each example, 1-5 and 6-10 represent serial dilutions of tissue samples; 1 or 6 = undiluted, 2 or 7 = 1:5 dilution, 3 or 8 = 1:20 dilution, 4 or 9 = 1:80 dilution, 5 or 10 = 1:320 dilution.

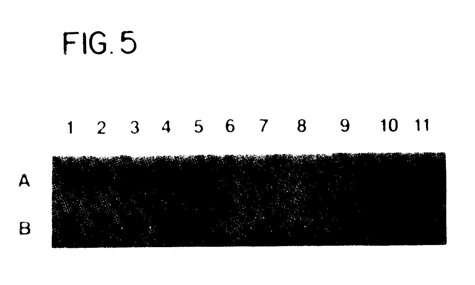

Fig. 5 shows the hybridization of radiolabeled cloned RA-1 parvovirus DNA with DNA contained in extracts of human synovial fluids from joints of patients with different types of arthritis. Purified $^{32}$P-labeled Hind III/Taq I 5 kb insert DNA from an RA-1 recombinant plasmid was used to assay DNA in phenol extracts of whole synovial fluids from individual patients with rheumatoid arthritis (specimens 1-5) or osteoarthritis (specimens 6-9). Positive controls shown are a phenol extract of DNA from Hirt supernates of RA-1 virus infected mouse brain tissue diluted 1:5 (specimen 10) and 1:100 (specimen 11), respectively.

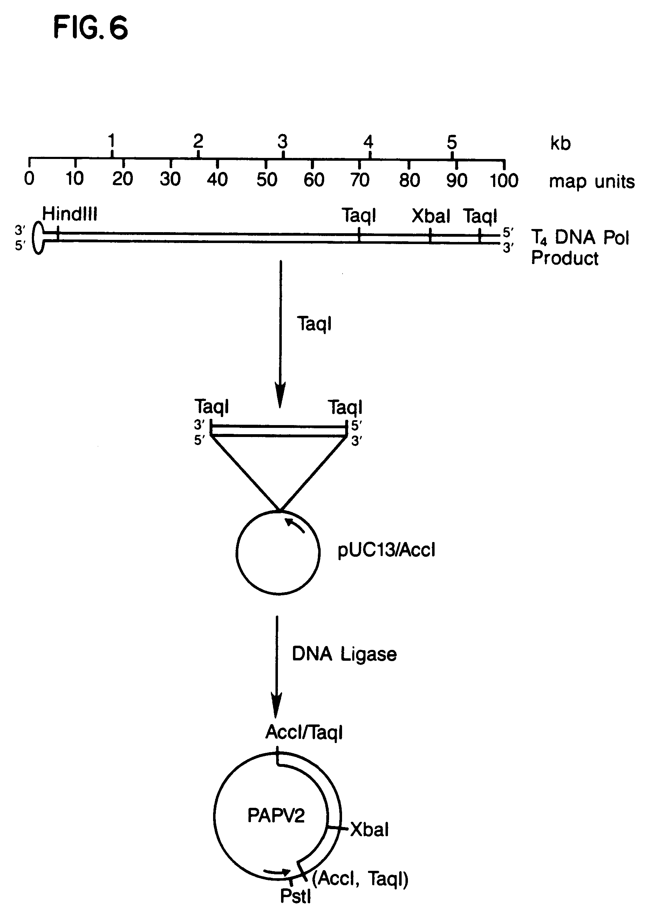

Fig. 6 illustrates the cloning of RA-1 DNA from map units 70 to 95 by digestion of the RA-1 in vitro DNA product with Taq I followed by ligation of the Taq I generated DNA fragments into pUC13. Plasmids containing RA-1 DNA were then screened for the presence of an internal Xba I site indentifying the 70 to 95 map unit segment.

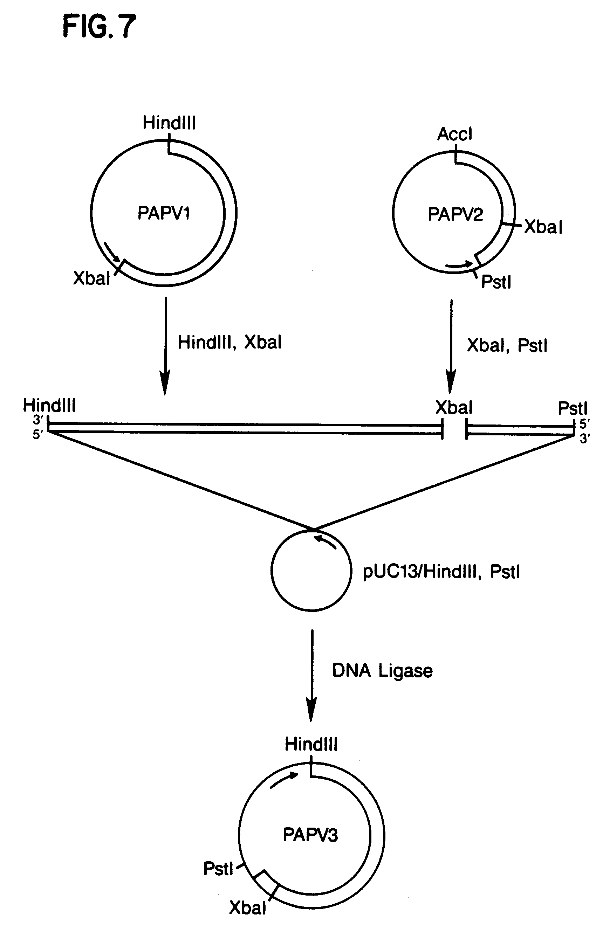

Fig. 7 shows the combination of RA-1 sequences contained within pAPV1 (5 to 85 map units) with pAPV2 (70 to 95 map units) to form pAPV3 (5 to 95 map units) containing the complete RA-1 coding region. RA-1 DNA was excised from pAPV1 by digestion with restriction endonucleases Hind III and Xba I and from pAPV2 with Xba I and Pst I. The two DNA segments were then ligated together into pUC13.

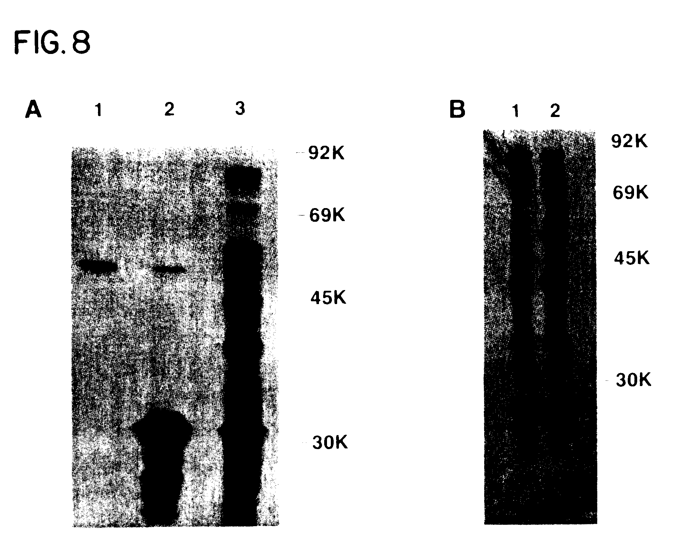

Fig. 8 demonstrates the expression of the RA-1 DNA coding region (5 to 95 map units) in vitro in an E. coli cell-free lysate, Panel A; and in vivo in protease-resistant E. coli strain WM6, Panel B; in comparison to the gene products of pUC13. Panel A: no DNA (lane 1), pUC13 (lane 2), pAPV3 (lane 3). Panel B: pUC13 (lane 1), pAPV3 (lane 2). Samples were resolved by SDS-polyacrylamide gel electrophoresis.

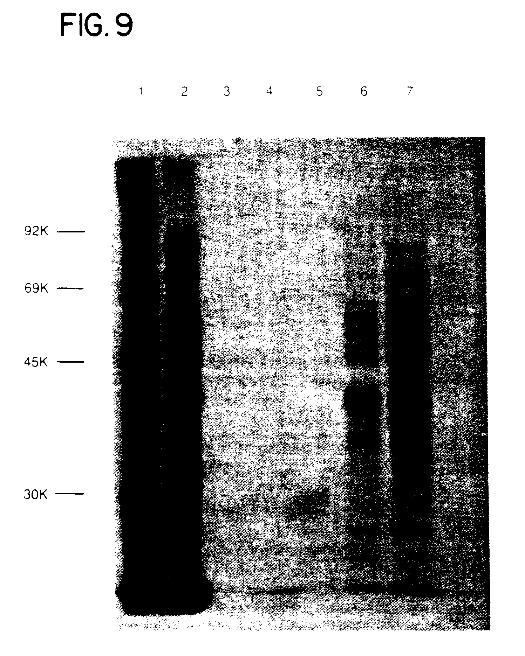

Fig. 9 shows the immune precipitation of parvovirus RA-1 gene products expressed in vitro in E. coli cell-free lysates. Depicted are the mobilities on SDS-polyacrylamide gel of protein molecular weight markers (lane 1), pAPV3 in vitro products (lane 2), blank (lane 3), immune precipitation of pAPV3 in vitro products by no antibody (lane 4), normal mouse serum (lane 5), RA-1 infected mouse serum #1 (lane 6), RA-1 infected mouse serum #2 (lane 7).

DETAILED DESCRIPTION OF THE INVENTION

Preferred embodiments of the invention will now be described. It will be understood by those skilled in the art that the description thereof is intended to be merely illustrative of the invention and not limitative thereof. For example, while specific methods and procedures describe the cloning and gene expression of parvovirus RA-1 DNA derived from single-stranded virion DNA, it is to be appreciated that double-stranded replicative intermediate forms of RA-1 DNA, chemically synthesized single or double-stranded genomic RA-1 DNA or portions thereof, or the DNA of other parvovirus strains related to RA-1 can also, for example, be cloned or function as DNA probe material, or can be used for synthesis of viral peptides, utilizing the inventive concepts hereof. In addition, while the particularly preferred embodiment described herein conducts DNA synthesis using T4 polymerase, other DNA polymerase materials may be used in the practice of the invention.

Reagents and Enzymes

Restriction endonucleases and T4 DNA polymerase were purchased from New England Biolabs. $^{32}$P-labeled radiochemicals, including nick-translation reagents, were purchased from New England Nuclear. $^{35}$S-labeled radiochemicals were purchased from Amersham. Agarose for gel electrophoresis was supplied by SeaKem. Acrylamide and other electrophoresis reagents were supplied by Bio Rad. Growth media were obtained from Difco.

Host Cell Cultures and Vectors

E. coli strains TB-1 and JM83 were obtained from Bethesda Research Laboratories and Pharmacia Molecular Biologicals, respectively. E. coli strain WM6 was developed and is available from Abbott Laboratories, North Chicago, Illinois as a derivative of CAG 456 which carries a nonsense mutation in the htpR (rpoD) gene encoding the RNA polymerase sigma subunit specific for heat shock promoters. The mutation may be suppressed, and proteolysis restored, by a temperature sensitive suppressor gene when cells are grown at low temperature (Baker, et al., Proc. Nat'l. Acad. Sci. (USA), 81:6779-6783 (1984)). Strain WM6 was isolated as a revertant of CAG 456 which can grow at higher temperatures, up to 42° C. The proteolytic defect is maintained in this WM6 strain. An advantageous feature of strain WM6 is the lack of a need for heat induction to reduce proteolysis and the potential for the accumulation of expressed protein over a longer period of time. E. coli cell-free lysates for in vitro expression were purchased from Amersham. Plasmids used for cloning and expression (pUC8, pUC12 and pUC13) were purchased from Pharmacia Molecular Biologicals. The other plasmid vectors described herein (pAPV1, pAPV2 and pAPV3) are considered to be part of the instant invention.

Methods Employed in Preferred Embodiments of the Present Invention

1. General Methods

Standard procedures were used for plasmid purification, transformation of E. coli, and small scale plasmid preparation (the procedures of Maniatis, T., et al., Molecular Cloning, Cold Spring Harbor Laboratory, Cold Spring Harbor, New York (1982)), and analyses of proteins in SDS-polyacrylamide gels (the procedure of Laemmli, U.K., Nature 227:680-685 (1970)). Restriction enzymes and DNA ligase were used according to the manufacturers' recommendations.

2. Propagation of RA-1 Virus in Mice

Litters of newborn, non-inbred Swiss-Webster mice from Perfection Breeders, Inc. (Douglassville, Pennsylvania) were used as a source of virus from infected brain tissue. Mice at the ages of between 24 hours to 3 days were given intracerebral injections of approximately 0.03 milliliter (ml) of RA-1 virus stock diluted in balanced salt solution (BSS). The mice were then sacrificed by $CO_2$ asphyxiation at about 4 to 5 days after inoculation, at which time they displayed signs of central nervous system disorder such as tremor and ataxia. Brain tissue was aseptically removed from each mouse after death and pooled. Tissue was either immediately extracted for virus or frozen at -80°C until needed.

3. Purification of RA-1 Virus Particles

Freshly collected or frozen brain tissue from newborn mice infected with RA-1 virus was extracted by a slight modification of the Hirt technique (Hirst, B., J. Mol. Biol. 26:365-369 (1967). Each gram of tissue was combined with 1 ml of Hirt buffer lacking sodium dodecyl sulfate (SDS), and gently homogenized either with or without a physical grinding agent such as alundum. To the homogenate were added 6.5 volumes of complete Hirt buffer containing 0.6% SDS, and the mixture was gently swirled until it became viscous from the released DNA. The mixture was then chilled on ice for about 30 minutes, after which sterile aqueous 5 Molar (M) NaCl solution was added to a final concentration of 1M. After chilling overnight on ice, the Hirt lysate was centrifuged in the cold for 30 minutes at between 12,000 and 17,000 g. The upper Hirt supernate fluid was collected; the supernate was then re-centrifuged if separation of visible particulate matter was observed not to be complete.

Virus particles were concentrated by centrifugation through dense solutions of sucrose. Typically, a 30 ml aliquot of Hirt supernate containing virus was layered on a 7 ml "cushion" of 30% sucrose in 20 (mM) Tris-HCl, pH 7.4, and 1 mM EDTA, and centrifuged in a Beckman-type Ti 60 rotor either for 5 hours at 33,000 rpm or overnight at 22,000 rpm. After removing the upper fluids by aspiration, the pelleted virus particles were resuspended in 1 ml of buffer containing 50 mM Tris-HCl, pH 7.4, and 10 mM MgCl$_2$ by passage through a tuberculin syringe with a 25 gauge 5/8 inch needle. These suspensions of concentrated virus particles were usually immediately processed for DNA extraction, although they were sometimes stored at temperatures of -70°C or lower before use.

4. Extraction and Purification of RA-1 Viral DNA

A mixture of DNase I and RNase A was added to purified virus particles at a final concentration of 50 micrograms each per milliliter (ug/ml), and incubation was carried out at 37°C for 60 minutes. Thereafter, stock solutions of the following substances were added at the final concentrations indicated: 50 mM EDTA, 0.15 M NaCl and 0.5% SDS. Proteinase K was then added at 50 ug/ml and incubated at 56°C for 30 minutes. The digest was extracted with an equal volume of 1:1 mixture of buffer-saturated phenol, chloroform and isoamyl alcohol (25:24:1). The aqueous phase was combined with 3 volumes of absolute ethanol and DNA was precipitated at -20°C. The DNA was sedimented by centrifugation, dried in a stream of nitrogen and dissolved in about 0.3 ml of TE buffer (10 mM Tris-HCl, pH 7.5 and 1 mM EDTA). NaCl was added to a final concentration of 0.2 M and the DNA was precipitated with ethanol at -70°C for 10 minutes. The DNA precipitate was dried with nitrogen and completely dissolved in 0.05 ml of TE buffer. The purified viral DNA was usually stored at -70°C.

5. Synthesis and Purification of RA-1 Double-stranded DNA as a Product of T4 Polymerase

The 3'-terminus of RA-1 single-stranded viral DNA served as a primer in in vitro DNA polymerase reaction mixtures for the synthesis of complementary DNA and formation of a double-stranded DNA complex. The template DNA was first denatured with 150 mM NaOH at 37°C for about 30 minutes and neutralized with 0.5 M Tris-HCl, pH 7.4, and 150 mM HCl. A typical in vitro reaction mixture (100 microliters (ul) volume) contained the following components listed in the order of addition: 8 ul denatured and neutralized RA-1 DNA (20 to 50 ng), 4 ul each of 2 mM dCTP, 2 mM dGTP, 2 mM dTTP and 0.2 mM dATP, 1 ul of [$^{35}$S]-dATP (500 Ci/mmol), 38 ul deionized distilled water and 5 units of phage T4 DNA polymerase. If a non-radioactive DNA product was desired, the dATP was substituted for [$^{35}$S]-dATP at 2 mM concentration. The reaction was carried out at 37°C for 60 minutes and stopped by addition of EDTA to 10 mM.

The product DNA was purified by addition of NaCl to 0.25 M followed by extraction with an equal volume of phenol, chloroform and isoamyl alcohol (25:24:1). A second extraction of the organic phase was done with an equal volume of TENS buffer (10 mM Tris-HCl, pH 7.5, 1 mM EDTA, 100 mM NaCl and 0.1% SDS) and the aqueous phases were pooled. The DNA was purified by column chromatography with Sephadex G-75 (Pharmacia) equilibrated with TEN buffer (10 mM Tris-HCl, pH 7.5, 1mM EDTA, 100 mM NaCl). The recovered DNA was further concentrated by ethanol precipitation and dissolved in an appropriate buffer for its subsequent use.

### 6. Preparation of Radiolabeled RA-1 Cloned DNA for Hybridization

Plasmid clones of RA-1 DNA were used for the preparation of radioactive nucleic acid probes. pAPV1 or restriction endonuclease fragments from pAPV1 (free of pUC12 sequences) were labeled by a standard "NICK TRANSLATION" procedure (Rigby, P.W.J., et al., J. Mol. Biol. 113:237 et seq. (1977)). A typical 100 ul reaction contained 1 microgram (ug) of the DNA to be labeled. Deoxynucleoside triphosphates were present at 40 micromoles (umol) with the exception of the radiolabeled nucleotide $^{32}$P-dCTP (3000 Curies per millimole (Ci/mmol), which was present at approximately 1 uM. For some labeling reactions all four nucleoside triphosphates were radiolabeled and included at a concentration of 1 mM. the concentrations of the DNAse I and E. coli DNA polymerase I used were adjusted to produce a population of labeled DNA products with an average size of 100 to 500 bases, as determined by gel electrophoresis under denaturing conditions. The specific activity of the labeled probe was approximately $5 \times 10^7$ counts per minute per microgram (cpm/ug) using one labeled nucleoside triphosphate.

### 7. Conditions for Electrophoresis of Parvovirus RA-1 DNA

Neutral agarose gel electrophoresis for the analysis of DNA was done in 0.7% agarose gels in a Tris-acetate buffer (40 mM Tris, 5 mM Na acetate, 2 mM EDTA, pH 7.9, with acetic acid). Ethidium bromide was included at 0.05 ug/ml. Alkaline agarose gel electrophoresis of denatured DNA was performed in 1.4% agarose gels in 50 mM NaOH and 2 mM EDTA. After electrophoresis, gels were neutralized and stained with ethidium bromide. Autoradiography of labeled DNA was accomplished by first drying gels onto DE81 paper (Whatman). DNA restriction fragments separated by gel electrophoresis were recovered from agarose gels by electroelution of the DNA bands from gel slices into dialysis bags filled with neutral gel buffer. DNA was recovered by concentration of the buffer by repeated extraction with sec-butanol followed by ethanol precipitation of the DNA. An alternate method to recover DNA from the electroelution buffer used Elutip-d-(TM) (Schleicher & Schuell) and the method recommended by the manufacturer.

### 8. Hybridization of Radiolabeled RA-1 DNA to DNA Derived from Mouse Tissues

Tissue extracts or cells were applied to a BA 85 nitrocellulose filter (Schleicher and Schuell) using a multiwell filtration manifold to concentrate the samples. After application of samples, the filter was placed sample side up on a piece of filter paper (Whatman 3MM), saturated with 0.5M NaOH, for 7 minutes. The nitrocellulose was transferred to pieces of filter paper saturated with 0.6 M NaCl and 1 M Tris, pH 6.8, to neutralize the alkali (twice 1 minute each), then transferred to a filter saturated with 1.5 M NaCl and 0.5M Tris, pH 7.4, for 5 minutes. The filter was then air dried, washed with 95% alcohol, washed twice in chloroform and rinsed in 0.3 M NaCl. After air drying the filter was baked at 80°C in a vacuum oven for 2-4 hours.

The baked nitrocellulose filter was wetted in 5X SSPE (90 mM NaCl, 50 mM NaPO4, pH 7.7, 5mM EDTA) then prehybridized in 5X SSPE with 5x Denhardts reagent (0.1% Ficoll, polyvinylpyrollidone and BSA) and 100 ug/ml denatured salmon sperm DNA for 2 hours at 65°C. The filter was then transferred to hybridization buffer (5X SSPE), containing 2X Denhardts, and 100 ug/ml denatured salmon sperm DNA. Denatured radiolabeled ($^{32}$P)- pAPV1 was included at 3 ng/ml. Hybridization was for 20 hours at 65°C. The filter, washed extensively at high stringency with several changes of buffer (0.1X SSPE, 0.1% SDS at room temperature) was then exposed to X-ray film (Kodak XAR-5) for 24 hours at -70°C.

### 9. Hybridization of Cloned RA-1 DNA Probes to DNA Contained in Clinical Specimens

Molecular DNA clones of RA-1 were used to screen for complementary nucleic acid sequences in clinical samples from humans with connective tissue disorders. For hybridization on solid nitrocellulose filters, synovial fluid was directly applied to a filter (wetted with 5X SSC buffer) and treated in situ with 0.5 M NaOH followed by neutralization (see previous section). Alternatively, the joint fluids were first treated with 100 mg/ml proteinase K in 0.1 M Tris-HCl, pH 8, and 0.5% SDS (55°C, 2 hours), then extracted with phenol, chloroform and isoamyl alcohol (25:24:1), to reduce or eliminate interferring substances and nonspecific binding of labeled probe. These nucleic acid extracts were treated with 0.3 M NaOH at 60°C for 60 minutes and neutralized with 0.5 M Tris-HCl, pH 7.4, before applying to nitrocellulose filters.

### 10. Prokaryotic DNA-directed Translation of Parvovirus RA-1 DNA

7

A prokaryotic DNA-directed translation kit (Amersham) was used to assay the expression of parvovirus RA-1 cloned DNA in vitro. The reactions were carried out according to the Amersham protocol using 1/2 reaction volumes. 0.3 uM DNA template and 10 uCi/ul of L-[35][S] methionine were used per reaction.

The samples were suspended in 2X Laemmli sample buffer and heated for 5 minutes at 90°C. One-fifth volume of the reaction products were resolved on a 10% SDS-PAGE gel. The gel was fixed for 30 minutes in 10% acetic acid and 40% methanol, treated 15 minutes with Enlightning (NEN), then dried down on filter paper and exposed to XAR-5 (Kodak) film at -70°C overnight with an intensifying screen.

### 11. Expression of Parvovirus RA-1 DNA in E. coli

Plasmid DNA was transformed into E. coli WM6 cells. A single colony was inoculated into 3 ml Luria broth plus ampicillin and grown to a 0.5 $OD_{600}$ at 37°C. The cells were pelleted 5 minutes at 6,000 rpm and resuspended in 0.5 ml of labeling media to give a concentration of 1.0 OD/ml. The labeling media consisted of 1X M9 salts (Maniatis, T., et al., Molecular Cloning, Cold Spring Harbor Laboratory, Cold Spring Harbor, N.Y., p. 440 et seq. (1982))), 10 mM $MgCl_2$, 0.001% vitamin $B_1$, 0.1 mM amino acids minus methionine, 2.4% glucose and 50 uCi L-[35][S] methionine. The cells were labeled for 30 minutes at 37°C, then pelleted and resuspended in 0.2 ml of 12.5 mM Tris-Cl (pH 7.5), 12.5% sucrose, 40 mM EDTA and 400 ug/ml lysozyme. The cells were next incubated on ice for 30 minutes followed by sonication for 15 seconds (0°C). 1 ul of the sonicate was run on a 10% SDS-PAGE gel and treated as described for in vitro translation.

### 12. Immune Precipitation of Parvovirus RA-1 Gene Products Expressed in E. coli Cell-free Lysates

#### a. Preparation of E. coli Lysate

A single colony of E. coli (JM103) cells containing pUC13 was inoculated into 5 ml of luria broth, 50 ug/ml ampicillin (L-AMP), and grown overnight at 37°C. A 1/50 dilution of the overnight culture in 25 ml L-AMP was grown to an $OD_{600}$ of 1.5. The cells were pelleted by centrifugation at 5,000 rpm for 10 minutes in an SS-34 rotor (4°C). The supernatant was decanted and the cells were resuspended in 0.75 ml of Buffer "A" (25% sucrose, 50 mM Tris, pH 8.0). The cells were then transferred to a 5 ml polystyrene tube and 0.15 ml of Buffer "B" (50 mM Tris, pH 8.0, 10 mM EDTA; containing 5 mg/ml lysozyme (Sigma)) was added. This mixture was incubated for 10 minutes on ice. 0.3 ml of Buffer "C" (0.25 M Tris, pH 8.0, 0.25 M EDTA) was added to the cells and the mixture incubated as before. Following the second incubation, 1.2 ml of Buffer "B" containing protease inhibitors (4 uM PMSF, 1.2% Aprotinin - Sigma) were added. The cells were then sonicated for 15-30 seconds on ice or until lysed, and stored at -20°C.

#### b. Preparation of Protein A Sepharose

Protein A sepharose CL-4B (Pharmacia) powder was hydrated in phosphate buffer following the Pharmacia protocol. An appropriate amount of the suspension was then centrifuged in an Eppendorf microfuge for 1 minute, after which the liquid was discarded and the pellet resupsended in 1/2 volume RIPA buffer (0.15 M NaCl, 1% deoxycholate, 1% triton X-100, 0.1% SDS, 10 mM Tris, pH 7.2) and 1/2 volume of E. coli lysate. This suspension was mixed for 15 minutes at 4°C. 50 ul of the pre-blocked protein A sepharose were used in each reaction.

#### c. Preparation of Antigen for Immune Precipitation

The antigen (2-5 ul of L-($^{35}$S)-met labelled products from an in vitro E. coli DNA-directed transcription/translation kit (Amersham) was treated by adding 50 ul of pre-blocked protein A sepharose and 100 ul RIPA buffer to each sample. The suspension was mixed for 15 minutes at 4°C before centrifuging for 1 minute in a microfuge. The supernatant was then removed to a new tube.

#### d. Preparation of Antisera and Immune Complex Formation

Antisera was also pre-blocked with the E. coli lysate. 5 ul of antisera and 15 ul of lysate were mixed for at least 2 hours at 4°C, then spun at 10,000 rpm for 10 minutes. The supernatant was added to the antigen supernatant (above) and incubated from 4 hours to overnight at 4°C.

e. Immune Precipitation and Washing Steps

After incubating the antigen-antibody mixture, 50 ul of pre-blocked protein A sepharose was added to each reaction and mixed for 15 minutes at 4°C. The mixture was spun for 1 minute in a microfuge at room temperature and the supernatant was removed. The pellet was washed with 3 successive 1 ml washes (Wash Buffer 1: 1 M NaCl, 10 mM Tris, pH 7.2, 0.1% NP-40; Wash Buffer 2: 0.1 M NaCl, 10 mM Tris, pH 7.2, 0.1% NP-40, 1mM EDTA, 0.3% SDS; Wash Buffer 3: 10 mM Tris, pH 7.2, 0.1% NP-40) followed by centrifugation for 1 minute at room temperature.

f. Gel Electrophoresis of Immune Precipated Protein Products

Following the washes, the pellet was resuspended in 20 ul of 2X Laemmli sample buffer, boiled for 5 minutes and run on a 10% Laemmli SDS-polyacrylamide gel. The gel was fixed in 40% methanol and, 10% acetic acid for 30 minutes, then treated for 15 minutes in Enlightning (NEN) and dried down on 3 MM Whatman filter paper. The gel was then exposed to Kodak XAR-5 film at 70°C with an intensifying screen.

ILLUSTRATIVE EXAMPLES

Example 1

Synthesis of RA-1 T4 DNA Polymerase Product

Purified DNA from RA-1 virus particles is a linear single stranded DNA thought to contain pallindromic sequences at each termini. The putative 3′-terminal pallindrome served as a primer for in vitro synthesis of DNA complementary to RA-1 viral DNA using T4 DNA polymerase (Fig. 1). The size of the double-stranded DNA products was determined to be about 5,300 base pairs by neutral agarose gel electrophoresis and 11,000 bases by alkaline agarose gel electrophoresis (Fig. 2). Purified double-stranded DNA product was subjected to digestion by restriction endonucleases and analysis by gel electrophoresis. A map of restriction sites within the RA-1 genome was then compiled (Fig. 3).

Example 2

Molecular Cloning of RA-1 DNA

The duplex DNA product of RA-1 viral DNA from the in vitro T4 DNA polymerase synthesis was the source of material for cloning the RA-1 genome. The product DNA was digested with restriction endonucleases Hind III and Xba I. These enzymes cut at sites near the ends of the product and generate a 4.3 kilobase DNA fragment (5 to 85 map units) which represents approximately 80% of the viral genome. RA-1 DNA was joined to the DNA of plasmid vector pUC12 cut with Hind III and Xba I using T4 DNA ligase to form recombinant plasmid pAPV1 (Fig. 1).

DNA from the ligation was introduced into E. coli strain TB-1 by a standard transformation procedure (see General Methods, supra). Cells were plated on media containing the antibiotic ampicillin and the lac Z indicator X-gal. Disruption of the lac Z region of pUC12 by the insertion of a restriction fragment between the Hind III and Xba I sites should produce a lac Z⁻ phenotype. Colonies that displayed the correct phenotype were cultured and screened for plasmid DNA.

Plasmid DNAs prepared from these strains were cut with restriction endonucleases Hind III and Xba I, and the DNA fragments generated were analyzed by agarose gel electrophoresis. Plasmids which contained a 4.3 kb fragment and a fragment of the same size as the parent plasmid pUC12 (2.7 kb) were designated pAPV1. That these clones represent RA-1 DNA was confirmed by restriction mapping and hybridization to DNA derived from RA-1 infected mouse tissues (Fig. 3 and Example 3).

Example 3

Hybridization of Cloned RA-1 DNA Nucleic Acid Probes to DNA From Normal and RA-1 Infected Mouse Tissues.

In order to confirm that plasmid pAPV1 contains genomic sequences of parvovirus RA-1, plasmid DNA was radiolabeled and used as a nucleic acid probe to screen for related nucleic acid sequences in extracts of normal and RA-1 infected mouse tissues applied to a nitrocellulose filter.

The autoradiograph of the filter (Fig. 4) shows that pAPV1-related nucleic acid sequences can be detected in Hirt extracts of RA-1 infected mouse brain tissue (positions C1-C5) and phenol extracts of these RA-1 infected mouse brain Hirt supernates (positions E6-E10). No related sequences were detected in Hirt extracts of normal mouse brain tissues or phenol extracts (positions A1-A5, E1-E5). On a longer exposure (20 hours), pAPV1 related DNA sequences were detected in Hirt extracts of RA-1 infected mouse liver (positions (C6-C10).

It is to be appreciated that the association of parvovirus RA-1 with human connective tissue disorders can be explored using similar DNA probe methodology in accordance with the present invention.

Example 4

Hybridization of Cloned RA-1 DNA Nucleic Acid Probes to DNA from Patients with Connective Tissue Disorders

DNA was extracted from the synovial fluids of patients diagnosed as having rheumatoid arthritis or osteoarthritis. The DNA was then applied to a nitrocellulose filter and annealed to $^{32}$P-labeled parvovirus RA-1 DNA probe derived from pAPV3 (see Example 6). DNA from RA-1 infected mouse brains was also applied as a positive control. The autoradiograph of the filter (Fig. 5) shows that DNA from patients with rheumatoid arthritis hybridized efficiently to RA-1 probe DNA as compared to DNA from RA-1 infected mouse brain tissue. By contrast, the DNA of osteoarthritis patients hybridized only weakly or hybridization was not detectable. This data suggests a positive correlation between the presence of parvovirus RA-1 DNA in human tissues and human rheumatoid arthritis. The data further demonstrates the usefullness of DNA probes procuded in accordance with the present invention as diagnostic reagents for screening clinical specimens for parvovirus RA-1.

Example 5

Construction of pAPV2

In order to synthesize polypeptides of parvovirus RA-1, it was necessary to first extend the cloned DNA of RA-1 toward the 5′-terminus. Comparison of the RA-1 T4 polymerase product DNA to published sequence data for MVM parvovirus suggested that the RA-1 cloned DNA in pAPV1 (5 to 85 map units) may be lacking the region coding for the C-terminus of structural polypeptides VP1 and VP2 ((Cotmore, S.F., et al., Virology 129:333-343 (1983)). To obtain the remaining coding region required for capsid gene expression, a second series of clones was constructed.

The restriction endonuclease Taq I cuts at four sites within RA-1 product DNA. The second largest restriction fragment, approximately 1500 base pairs, represents a region of the genome from 70 to 95 map units which should contain the remaining coding region for the C-terminus of VP1, VP2. This Taq I fragment was cloned into plasmid vector pUC13 to generate clone pAPV2 (Fig. 6). DNA from a Taq I digest of product DNA was ligated to Acc I linearized pUC13. Transformation of E. coli TB-I with the ligation product yielded many recombinant clones with the correct phenotype (Amp, $\overline{lacZ^-}$). To identify the clone which contained the desired Taq I fragment, plasmid DNAs from the clones were cut with the restriction enzyme Xba I. Only clones containing the second largest Taq I fragment should contain an Xba I site within the insert. A clone with the correct restriction endonuclease map was designated pAPV2.

Example 6

Construction of pAPV3

Recombinant plasmid pAPV3 is a molecular clone of the region of the RA-1 genome from 5 to 95 map units. The palindromic regions from the termini of the viral DNA are not represented, but all of the coding regions for the viral proteins should be present. pAPV3 was constructed by combining restriction enzyme fragments from pAPV1 and pAPV2 with plasmid vector pUC13 (Fig. 7). The 4.3 kilobase fragment from a Hind III and Xba I digest of pAPV1 represents the region of the RA-1 genome from 5 to 85 map units. pAPV2 digested with Xba 1 and Pst I generates a 600 base pair DNA fragment which represents 85 to 95 map units of the genome of RA-1. These two DNA fragments were purified by gel electrophoresis followed by electroelution. Both fragments were ligated with plasmid vector pUC13 cut with Hind III and Pst I. The ligation reaction product was used to transform E. coli strain JM83. Colonies grown on selective media that demonstrated the correct phenotype (Amp$^R$, lacZ$^-$) were screened for the presence of plasmid DNA. Plasmids that had a restriction map consistent with the predicted recombinant were designated pAPV3.

Example 7

Expression of parvovirus RA-1 gene products in E. coli

Plasmid pAPV3 is thought to contain the entire coding region of the RA-1 genome (5 to 95 map units) based on comparison to parvovirus MVM (Pintel, D., et al., Nucleic Acids Research, 11:1019-1038 (1983) and Cotmore, S.F., et al., Virology, 129:333-343 (1983)). The expressed gene products of pAPV3 were compared to the parent plasmid pUC13 in vitro in E. coli cell-free lysates (Fig. 8a) and in vivo in the protease- deficient E. coli strain WM6 (Fig. 8b). Expression of pAPV3 in vitro yielded four major proteins (91K, 65K, 50K, 39K) not present in pUC13 as well as a number of minor species. Analysis of expression products of pAPV3 in vivo showed that two proteins were present (38K, 31K) in addition to those observed from expression of pUC13.

Example 8

Immune Precipitation of Parvovirus RA-1 Gene Products Synthesized in vitro in E. coli

Products of parvovirus RA-1 DNA (pAPV3) expressed in an E. coli cell-free lysate were characterized by SDS-polyacrylamide gel electrophoresis. The antigenicity of the expressed proteins was determined by an immune precipitation assay using antisera from uninfected and RA-1 infected mice. Resolution of immune precipitated proteins by SDS-polyacrylamide gel electrophoresis is shown in Fig. 9. All of the polypeptides derived from in vitro expression of RA-1 DNA were immune precipitated by antisera of two RA-1 infected mice, but not in the absence of antiserum of with normal mouse antiserum.

Example 9

ELISA Assay for Parvovirus RA-1

Antigen was prepared as described in Example 7. The antigens were the gene products of pAPV3/WM6 and pUC8*/WM6 added to host cell cultures and vectors (pUC8* is pUC8 with a frame shift in the lac Z region). Immune reagents used were mouse anti-RA-1 polyclonal sera, normal mouse sera, and PBS buffer without serum.

Four dilutions of each antigen in triplicate samples were bound to a 96 well immuno plate (NUNC). Dilutions were made in PBS (phosphate buffered saline - Gibco). The plate was covered with a plate sealer (Dynatech) and incubated for 2 hours at 37°C. The plate was washed 5 times with PBS, and then residual buffer was shaken out of the plate. 200 ul of 2% BSA (Sigma Chemical Company) in PBS were dispensed in each well. The plate was covered and then incubated for 1 hour at 37°C. The plate was washed as before.

Immune reagents were diluted 1:100 with 0.1% BSA in PBS. 100 ul of the antisera dilution were added to each well. The plate was covered and incubated for 1 hour at 37°C. The plate was washed as before.

Horseradish peroxidase (HRPO) conjugated rabbit anti-mouse IgG (Cappel) at a 1:1000 dilution with 0.1% BSA in PBS was added to each well. The plate was covered and incubated for 1 hour at 37°C. The plate was again washed as before. 100 ul of o-phenylenediamine (OPD) substrate (one tablet of Abbott OPD substrate from an Abbott reagent kit in 10 ml of kit diluent) was added to each well and color was allowed to develop for a minimum of 10 minutes at room temperature. 100 ul of OPD solution was dispensed in a blank well as a control. The reactions were quenched with 100 ul of 1N $H_2SO_4$. The reactions were read at an optical density of 490 nm with a 405 nm filter in an immunoreader (Intermed NJ-2000).

Data from the assays were compiled by averaging the three duplicate readings which gave the highest ratio of pAPV3 antigenic reactivity relative to the pUC8* control (1:10 for both APV3/WM6 and pUC8*/WM6). As shown in Table I, the products of pAPV3 gave a signal 2-fold greater than the pUC8* control using antisera from RA-1 infected mice. By contrast, a similar (and lower) signal was seen for both the products of pAPV3 and pUC8* using normal mouse sera or in the absence of sera.

## TABLE I

|         | RA-1<br>Mouse Sera | Normal<br>Mouse Sera | No<br>Mouse Sera |
|---------|--------------------|----------------------|------------------|
| pAPV3   | 0.85               | 0.20                 | 0.10             |
| pUC8*   | 0.40               | 0.20                 | 0.12             |

These data show that the products of pAPV3 expressed in E. coli strain WM6 are antigenic. The expressed gene products are also useful as reagents in diagnostic assays for the determination of markers of RA-1 parvovirus in biological samples.

The American Type Culture Collection (ATCC) designation for the parvovirus RA-1 material described herein is VR2111.

It will be apparent to one skilled in the art to which the present invention pertains that various modifications and variations of the specific embodiments described herein can be made, without departing from the spirit and scope of the invention, as defined solely by the following claims.

**Claims**

1. Synthetic parvovirus RA-1 DNA derived from the parvovirus RA-1 DNA which when characterized by digestion with the restriction endonucleases Acc I, Bgl I, Bgl II, Eco RV, Hae II, Hinc II, Hind III, Pst I, Sac I, Sph I, Stu I and Xba I has the following restriction map

2. Synthetic parvovirus RA-1 DNA according to Claim 1, which has been made double-stranded using DNA polymerase.

3. Synthetic parvovirus RA-1 DNA according to Claim 2 inserted into a plasmid vector in E. coli.

4. A protein expression product, recombinantly produced in vitro in an expression system selected from the group consisting of E. coli, yeast, mammalian cells and nuclear polyhedrosis virus-insect cells, and derived from RA-1 DNA of Claim 1, said protein being an antigen of parvovirus RA-1.

5. A protein expression product, recombinantly produced in vivo in an expression system selected from the group consisting of E. coli, yeast, mammalian cells and nuclear polyhedrosis virus-insect cells, and derived from RA-1 DNA of Claim 1, said protein being an antigen of parvovirus RA-1.

6. An assay for parvovirus RA-1 which assay comprises utilizing a DNA probe comprising synthetic parvovirus RA-1 DNA of Claim 1.

7. The assay of Claim 6 wherein said synthetic parvovirus RA-1 DNA is derived from a plasmid vector cloned in E. coli.

**8.** An assay for detection of parvovirus RA-1 which assay utilizes the proteins of Claims 4 or 5.

**9.** An assay for determining the presence or absence of antibodies to parvovirus RA-1 in a sample comprising contacting the sample with the parvovirus RA-1 antigen of Claims 4 or 5 to form a reaction product, and then determining the presence or amount of said reaction product.

**10.** The assay of Claim 9 wherein said antigen is bound to a solid support and wherein the presence or amount of the reaction product is determined by contacting the reaction product with a second antibody capable of specifically binding the reaction product, said second antibody being conjugated to a label, and then detecting the presence of the label.

**Patentansprüche**

**1.** Synthetischer Parvovirus RA-1DNA, der sich vom Parvovirus RA-1 DNA, der bei Charakterisierung durch Verdauung mit den Restriktionsendonukleasen Acc I, Bgl I, Bgl II, Eco Rv, Hae II, Hinc II, Hind III, Pst I, Sac I, Sph I, Stu I und Xba I die folgende Restriktionskarte zeigt, ableitet.

**2.** Synthetischer Parvovirus nach Anspruch 1, der unter Verwendung von DNA-Polymerase doppelsträngig hergestellt wurde.

**3.** Synthetischer Parvovirus nach Anspruch 2, der in einen Plasmid-Vektor in E. coli eingeführt wurde.

**4.** Protein-Expressionsprodukt, das in vitro in einem Expressionssystem ausgewählt aus der Gruppe bestehend aus E. coli, Hefe, Säugetier-Zellen und nuklearen Polyhedrose Virus-Insektenzellen, rekombinant hergestellt wurde, und das sich von RA-1 DNA nach Anspruch 1 ableitet, wobei das genannte Protein ein Antigen des Parvovirus RA-1 ist.

**5.** Protein-Expressionsprodukt, das in vivo in einem Expressionssystem ausgewählt aus der Gruppe bestehend aus E. coli, Hefe, Säugetier-Zellen und nuklearen Polyhedrose Virus-Insektenzellen, rekombinant hergestellt wurde, und das sich von RA-1 DNA nach Anspruch 1 ableitet, wobei das genannte Protein ein Antigen des Parovirus RA-1 ist.

**6.** Test auf Parvovirus RA-1, wobei der Test die Verwendung einer DNA-Sonde, die den synthetischen Parvovirus RA-1 DNA nach Anspruch 1 umfaßt, umfaßt.

**7.** Test nach Anspruch 6, wobei sich der genannte synthetische Parvovirus RA-1 DNA von einem in E. coli klonierten Plasmid-Vektor ableitet.

**8.** Test zur Detektion des Parvovirus RA-1 DNA, wobei der Test die Proteine nach Anspruch 4 oder 5 verwendet.

**9.** Test zur Bestimmung von Anwesenheit oder Abwesenheit von Antikörpern gegen Parvovirus RA-1 DNA in einer Probe, die die Behandlung der Probe mit dem Parvovirus RA-1 DNA-Antigen nach Anspruch 4 oder 5 zur Bildung eines Reaktionsproduktes umfäßt, und dann zur Bestimmung der Anwesenheit oder der Menge des genannten Reaktionsprodukts.

**10.** Test nach Anspruch 9, wobei das genannte Antigen an eine feste Stütze gebunden ist und worin die Anwesenheit oder Menge des Reaktionsprodukts durch Behandlung des Reaktionsprodukts mit einem zweiten Antikörper, der zur spezifischen Bindung des Reaktionsprodukts fähig ist, bestimmt wird, wobei der genannte zweite Antikörper paarweise an eine Marker gebunden ist und dann in Gegenwart des Markers detektiert wird.

**Revendications**

1. ADN synthétique du parvovirus RA-1 dérivé de l'ADN du parvovirus RA-1 qui lorsqu'il est caractérisé par une digestion avec les endonucléases de restriction Acc I, Bgl I, Bgl II, Eco RV, Hae II, Hinc II, Hind III, Pst I, Sac I, Sph I, Stu I et Xba I a la carte de restriction suivante

2. ADN synthétique du parvovirus RA-1 selon la Revendication 1, qui a été fait double brin en utilisant une polymérase d'ADN.

3. ADN synthétique du parvovirus RA-1 selon la Revendication 2 inséré dans un vecteur plasmidique dans E.coli.

4. Un produit d'expression protéique, produit par recombinaison in vitro dans un système d'expression choisi dans le goupe constitué par E.coli, levure, cellules de mammifères et cellules d'insectes infectées par le virus de la polyhédrose nucléaire, et dérivé de l'ADN de RA-1 de la Revendication 1, ladite protéine étant un antigène du parvovirus RA-1.

5. Un produit d'expression protéique, produit par recombinaison in vivo dans un système d'expression choisi dans le groupe comprenant E.coli, levure, cellules de mammifères et cellules d'insectes infectées par le virus de la polyhedrose nucléaire, et dérivé de l'ADN de RA-1 de la Revendication 1, ladite protéine étant un antigène du parvovirus RA-1.

6. Un dosage pour le parvovirus RA-1 lequel dosage comprend l'utilisation d'une sonde d'ADN comprenant de l'ADN synthétique du parvovirus RA-1 de la Revendication 1.

7. Le dosage de la Revendication 6 dans lequel ledit ADN synthétique du parvovirus RA-1 est dérivé d'un vecteur plasmidique cloné dans E.coli.

8. Un dosage pour la détection du parvovirus RA-1 lequel dosage utilise les protéines des Revendications 4 ou 5.

9. Un dosage pour déterminer la présence ou l'absence des anticorps contre le parvovirus RA-1 dans un échantillon consistant à mettre l'échantillon en contact avec l'antigène du parvovirus RA-1 des Revendications 4 ou 5 pour former un produit de réaction, et ensuite à déterminer la présence ou la quantité de ce produit de réaction.

10. Le dosage de la Revendication 9 dans lequel ledit antigène est lié à un support solide et dans lequel la présence ou la quantité du produit de réaction est déterminée en mettant en contact le produit de la reaction avec un second anticorps capable de fixer spécifiquement le produit de la réaction, ledit second anticorps étant conjugué à un marqueur, et ensuite en détectant la présence du marqueur.

# FIG. 1

# FIG. 2

A  1 2 3    B  1 2 3

# FIG.3

**NUCLEOTIDES**

**MAP UNITS**
**Non-Cutters:** AvaI, AhaIII, BalI, BamHI, ClaI, EcoRI,
KpnI, SalI, SmaI, XhoI

EP 0 238 893 B1

FIG. 4

# FIG. 5

1  2  3  4  5  6  7  8  9  10  11

A

B

# FIG. 6

# FIG.7

FIG.8

FIG. 9